# NOUVEAU FASCICULE DE BREVET EUROPEEN

(11) **EP 0 359 593 B2**
(45) Date de publication et mention de la décision concernant l'opposition: **07.01.2004**
(45) Mention de la délivrance du brevet: 26.04.1995
(21) Numéro de dépôt: 89400348.2
(22) Date de dépôt: 08.02.1989
(51) Int. Cl.: C07K 1/00, C07K 2/00, A61K 35/16

(54) **Séparation chromatographique des protéines du plasma, notamment du facteur VIII, du facteur von Willebrand, de la fibronectine et du fibrinogène**
Chromatographische Trennung von Plasmaproteinen, insbesondere von Faktor VIII, von Willebrand Faktor, von Fibronectin und von Fibrinogen
Chromatographic separation of plasma proteins, particularly of factor VIII, of von Willebrand factor, of fibronectin and of fibrinogen

(30) Priorité: 07.06.1988 FR 8807530
(43) Date de publication de la demande: 21.03.1990
(73) Titulaire: CENTRE REGIONAL DE TRANSFUSION SANGUINE DE LILLE, 59012 Lille (FR)
(72) Inventeur: Burnouf, Thierry, F-59136 Wavrin (FR); Burnouf Myriana, F-59136 Wavrin (FR)
(74) Mandataire: Lepeudry-Gautherat, Thérèse

(56) Documents cités:
- EP-A- 343 275
- EP-A- 0 176 926
- EP-A- 0 237 981
- EP-A- 0 317 376
- WO-A-86/04486
- REAGENTS MERCK, "Fractogel (R) TSK", Polymeric Media for Biochromatography#
- Fractogel TSK "Polymeric Media for Biochromatography Reagents Merck//

## Description

L'invention concerne la séparation de protéines d'une fraction de plasma humain ou animal par chromatographie d'échange d'anions selon une technique permettant d'obtenir en une seule étape un taux de purification très important, notamment du Facteur VIII, du fibrinogène et du facteur von Willebrand.

La mise à disposition de protéines du sang nécessite, pour leur utilisation à des fins thérapeutiques, des techniques de purification permettant d'obtenir des produits de haute pureté et totalement dépourvus de contaminants, notamment d'autres protéines ou de substances d'origine étrangère telles que des anticorps.

Ainsi, il est essentiel, dans le traitement de l'hémophilie A, de disposer de concentrés de Facteur VIII de très haute pureté en effet, les malades subissent des injections nombreuses et répétées de concentrés de Facteur VIII et, simultanément, de quantités importantes de fibrinogène et d'immunoglobulines qui peuvent induire des réponses immunitaires indésirables. Ainsi des injections répétées de Facteur VIII insuffisamment purifié ne peuvent être réalisées qu'avec des concentrés de plasmas isogroupes pour éviter les accidents typiques des transfusions dûs aux différences de groupes sanguins et provoqués par la présence d'immunoglobulines.

Les concentrés de Facteur VIII sont le plus souvent préparés à partir d'une fraction de plasma humain cryoprécipité. La pureté des concentrés de Facteur VIII généralement obtenus dans les centres industriels de traitement du plasma humain est souvent de l'ordre de 1 UI/mg et n'excède généralement pas les limites de 10 à 20 UI/mg. Les techniques de production classique font appel à des étapes de précipitation qui visent à éliminer, souvent très imparfaitement, les contaminants protéiques tels que le fibrinogène, la fibronectine, et les immunoglobulines. Ces techniques peuvent utiliser ou combiner une précipitation à faible température (10°C), ou l'adjonction d'agents de précipitation des protéines ; ainsi des polymères hydrophiles tels que le PEG (Newman et al., Br. J. Haematol 21:1-20, 1971 ; Hao et al., in Methods of Plasma Protein Fractionation,Academic Press 1980, pp.57-74), la polyvinylpyrrolidone (Casillas et Simonetti, Br. J. Haemato, 50:665-672, 1982), le dextran, le Ficoll, le Percoll, l'amidon hydroxyéthylé et l'albumine ont été proposés comme agents de précipitation du Facteur VIII (Farrugia et coll., Thromb Haemostas, 51:338-342,1984). Il en est de même de l'usage de glycocolle et de chlorure de sodium préconisé par Thorell et Blömback. De la même façon, certains auteurs (Ng et al, Thrombosis Res.,42:825-834, 1986) ont réussi à combiner trois agents de précipitation qui sont le PEG, le glycocolle, et le chlorure de sodium pour obtenir des concentrés de Facteur VIII à activité spécifique comprise entre 10 et 16 UI/mg.

On a fait appel aussi à des techniques de chromatographie d'exclusion stérique, ou tamisage moléculaire, qui visent à la récupération d'une fraction de haut poids moléculaire contenant le complexe Facteur VIII:C-facteur von Willebrand partiellement dépourvu de fibrinogène. Cette technique fournit à faible rendement un produit dont l'activité spécifique ne dépasse pas 30 UI/mg et qui requiert l'adjonction d'albumine comme stabilisant (conduisant à un abaissement de l'activité spécifique à environ 3 à 5 UI/mg). Cette technique pose quelques problèmes d'adaptation à grande échelle car il est difficile de maintenir constant dans le temps le pouvoir de résolution des colonnes industrielles de tamisage moléculaire.

Des concentrés de Facteur VIII ont été produits également en intégrant au protocole de production un contact avec des billes de silice poreuse destinées à emprisonner les contaminants protéiques de faible poids moléculaire (Margolis et al., Vox Sang. 46:341-348,1984). L'activité spécifique du produit reste relativement faible : 1Ul/mg.

Des techniques nouvelles sont récemment apparues dans la préparation de concentrés de Facteur VIII de très haute pureté. Ainsi les firmes Hyland et Travenol ont elles proposé des concentrés obtenus par des méthodes de chromatographie d'immunoaffinité (Zimmerman et Fulcher, Thrombosis Res., Suppl. VII, p. 58, 1987 ; Berntorp et Nilsson, Thrombosis Res., Suppl. VII, p.60, 1987 ; Levine et al., Thombosis Res, Suppl. VII, 1987). Ces techniques consistent à purifier le Facteur VIII à l'aide d'anticorps anti-Facteur VIII:C ou anti-facteur von Willebrand immobilisés sur un support chromatographique. Ces techniques sont performantes mais exigent l'emploi de solutions drastiques pour désorber le Facteur VIII soit de son anticorps soit du facteur von Willebrand. Une étape supplémentaire d'ultrafiltration visant à éliminer les agents chimiques indésirables est donc nécessaire mais elle peut nuire à l'activité biologique du Facteur VIII. L'activité spécifique du Facteur VIII peut atteindre 1000 à 3000 UI/mg en cours de production, mais sa fragilité exige l'adjonction d'un stabilisant, tel que l'albumine, avant l'étape de lyophilisation, ce qui réduit l'activité spécifique du Facteur VIII à 3 à 5 UI/mg. L'inconvénient majeur de la purification par immunoaffinité est néanmoins la présence d'anticorps résiduels ; ceux-ci étant d'origine murine, ils peuvent entraîner chez les malades l'apparition de réactions immunologiques vis-à-vis de ces protéines étrangères à l'organisme humain.

On a également utilisé des techniques de chromatographie d'échange d'ions, mais compte tenu de la complexité des processus opératoires et des faibles rendements obtenus, ces techniques sont restées au stade du laboratoire.

Ainsi l'article de J.J.Morgenthaler (Thromb. Haemostas., Stuttgart, 47(2) 124 - 127 (1982) étudie la purification de Facteur VIII:C à partir de précipité au polyéthylèneglycol par passage sur une résine de Sepharose modifiée. Aucune indication n'est donnée sur le rendement et la reproductibilité des différentes techniques testées.

Il est donc tout à fait essentiel de disposer de nouvelles méthodes d'obtention de concentrés de protéines, et notamment de Facteur VIII, applicables à l'échelle industrielle et donnant des produits de très haute pureté, totalement dépourvus de protéines d'origine étrangère telles que des anticorps d'origine animale.

La demande de brevet européen n° 88108458.6 publiée le 29 novembre 1989 décrit un procédé de préparation de Facteur VIII à partir d'un cryoprécipité qui se caractérise en ce que, précédemment au traitement d'inactivation virale, on extrait le cryoprécipité décongelé par de l'eau contenant 1 à 3 U/ml d'héparine à pH 6,5-7,5 on fait réagir avec une suspension d'hydroxyde d'aluminium et après refroidissement à 10-18°C et réglage du pH à 6-7, on centrifuge ou on filtre puis on poursuit la purification par un traitement ultérieur, en particulier par chromatographie de perméation de gel sur une résine échangeuse d'ions telle que Fractogel-DEAE, de type hydrophile.

Ce document décrit donc la purification du seul Facteur VIII par l'association d'une étape préliminaire très particulière, se caractérisant notamment par l'absence totale de traitement éthanolique, avec une chromatographie d'échange d'ions sur une résine hydrophile telle que Fractogel DEAE.

La Demanderesse a maintenant mis au point un procédé de purification par chromatographie d'échange d'anions, qui, grâce à un choix judicieux de la résine utilisée, permet de séparer sur une seule colonne chromatographique les protéines recherchées dans des conditions suffisamment ménagées pour rendre inutiles les traitements ultérieurs, tels que l'ultrafiltration, qui augmentent la complexité du procédé et diminuent l'activité de la protéine purifiée.

L'invention concerne donc un procédé de séparation des protéines Facteur VIII, fibrinogène, fibronectine et de facteur von Willebrand du plasma humain ou animal, caractérisé en ce qu'on soumet une fraction resolubilisée dans l'eau d'un cryoprécipité de plasma humain à une séparation unique par voie chromatographique sur une résine échangeuse d'anions dont la matrice est un gel de type polymère vinylique macroréticulé capable de par ses propriétés de porosité et d'hydrophobicité de retenir le complexe Facteur VIII-facteur von Willebrand et qu'on récupère sélectivement les différentes protéines par des augmentations successives de la force ionique du tampon d'élution, selon la revendication 1.

Le procédé est également applicable à un plasma d'origine animale, par exemple porcine, pour les cas particuliers de malades hémophiles à sérum inhibiteur qui ne peuvent pas être traités avec du Facteur VIII d'origine humaine.

On peut ainsi utiliser comme fraction de départ une fraction de plasma cryoprécipité ayant éventuellement subi un traitement de pré-purification. Ce traitement préalable peut consister par exemple en une précipitation au gel d'alumine et/ou une précipitation à basse température selon les techniques classiques de traitement de telles fractions.

Parmi les différentes résines testées pour la séparation chromatographique, on a observé que les résultats les plus satisfaisants étaient obtenus en utilisant des groupements DEAE greffés sur une matrice présentant les propriétés indiquées ci-dessus, nécessaires à la fixation du complexe Facteur VIII-Facteur von Willebrand. Une résine de ce type est disponible dans le commerce sous l'appellation Fractogel® TSK-DEAE (Merck).

La Demanderesse a en effet constaté que ce type de résine, connu pour ses propriétés hydrophiles comme cela est rappelé dans la demande de brevet n ° 88 108458.6, possédait en fait également, dans des conditions de mise en oeuvre appropriées, des caractéristiques d'hydrophobicité suffisantes pour pouvoir retenir le complexe Facteur VIII-facteur von Willebrand conformément au procédé de la présente invention.

La résine Fractogel ® TSK-DEAE 650 de porosité de type M a ainsi fourni des résultats nettement supérieurs aux autres gels testés, comme la DEAE-Sépharose® CL-6B, DEAE-Sépharose CL-6B Fast-Flow (Pharmacia), DEAE-Sépharose 4B ou la DEAE-Trisacryl LS (IBF).

L'utilisation d'un gel semblable au Fractogel-TSK-DEAE(M) a été décrite par Y. Kato et al. (J. Chromato. 245,1982,193-211) et recommandée pour des séparations chromatographiques à moyenne performance et à l'échelle industrielle de proteines de très grande taille. La capacité de rétention-élution de ce gel macroréticulé repose sur un faible pouvoir d'échange ionique et sur la dimension importante des pores.

Ainsi la Demanderesse a montré que ce type de gel permettait d'adsorber préférentiellement les complexes de très grande taille qui se forment entre le Facteur VIII et le facteur von Willebrand. De plus, la Demanderesse a, par le choix des tampons d'équilibrage et d'élution, tiré parti de liaisons faiblement hydrophobes qui s'établissent grâce à la rétention prolongée des complexes de grande taille sur le support polyvinylique. Cet avantage du gel n'avait pas été mis en évidence précédemment.

En utilisant une telle résine pour traiter une fraction de plasma contenant du Facteur VIII, par exemple une solution pré-purifiée du cryoprécipité, on obtient, dans des conditions de tampon appropriées, un concentré de Facteur VIII de très haute pureté, et de qualité assimilable à celle d'un concentré de plasma isogroupe, à une concentration de l'ordre de 50 UI/ml (activité spécifique supérieure à 100 UI/mg) sans avoir à recourir à une étape d'ultrafiltration, et de grande stabilité,c'est-à-dire qui ne nécessite pas l'adjonction d'un stabilisant de nature protéique. On obtient aussi, par la même chromatographie, des fractions enrichies en fibrinogène, en fibronectine et en facteur von Willebrand, répondant aux paramètres physico-chimiques satisfaisant à un usage thérapeutique ou à titre de réactif. De plus le fibrinogène peut être concentré davantage pour être utilisé comme colle biologique, selon la demande de brevet européenne 88.401961.3.

La mise en oeuvre du procédé selon l'invention s'effectue comme suit. La fraction de plasma prépurifiée et contenant les protéines majeures du cryoprécipité, c'est-à-dire le fibrinogène, le Facteur VIII, la fibronectine et le facteur von Willebrand, passe sur une résine échangeuse d'anions telle que spécifiée ci-dessus ; le Facteur VIII, le facteur von Willebrand (la totalité ou une grande partie, selon la quantité de matériel de départ) et la fibronectine s'adsorbent sur la résine tandis que le fibrinogène se retrouve dans le filtrat des protéines non adsorbées.

Par une première augmentation de la force ionique du tampon on élue la fibronectine et une grande partie du facteur von Willebrand.

Par une augmentation supplémentaire de la force ionique du tampon le Facteur VIII s'élue en présence de faibles quantités de facteur von Willebrand et peut être directement lyophilisé sans qu'il soit nécessaire de lui ajouter un stabilisant ou de le soumettre à une étape d'ultrafiltration.

Le tampon utilisé contient avantageusement de la lysine à une dose de l'ordre de 2 à 4 g/l ainsi que du glycocolle à une dose de l'ordre de 8 à 11 g/l. L'utilisation d'autres acides aminés ou même de l'un seulement de ces deux acides aminés donne des résultats nettement moins satisfaisants.

L'augmentation de la force ionique du tampon se fait par adjonction de chlorure de sodium. En effet, l'utilisation unique de cette résine de caractère ionique modéré et de nature légèrement hydrophobe permet d'utiliser ce sel pour désorber le facteur von Willebrand avant l'élution du Facteur VIII ce qui évite le recours au chlorure de calcium, qu'il serait ensuite nécessaire d'éliminer par ultrafiltration,pour dissocier Facteur VIII:C et facteur von Willebrand.

On peut bien entendu prévoir un traitement d'inactivation virale selon une technique connue, à un stade quelconque du procédé. Dans le cas où on utilise un agent chimique d'inactivation virale, il sera judicieux d'effectuer cette inactivation juste avant le passage de la fraction de plasma sur la résine. De cette façon l'étape chromatographique servira à l'élimination efficace des agents d'inactivation.

On a obtenu de bons résultats en utilisant la technique d'inactivation par solvant-détergent, telle que décrite dans la demande de brevet européen No 0 131 740.

Pour la préparation de Facteur VIII l'étape de chromatographie peut être réalisée sur toute fraction protéique le contenant, par exemple sur une fraction plasmatique cryoprécipitée prépurifiée qui a été soumise à un traitement au gel d'alumine éventuellement suivi d'une précipitation à basse température, selon des méthodes classiques de production de concentrés de Facteur VIII, qui ont été décrites dans la demande de brevet européen EP-A-0 238 701.

L'activité spécifique du Facteur VIII:C dans la fraction de départ peut être aussi basse que 0,1 UI/mg. Le facteur de purification obtenu par une seule étape de chromatographie d'échange d'anions est selon l'invention de l'ordre de 400 à 700 fois. Le rendement de cette étape est compris entre 75 et 90%.

Le concentré de Facteur VIII obtenu est de très haute pureté, son activité spécifique étant supérieure à 100 UI/mg de protéines. Il est dépourvu de fibrinogène et d'immunoglobulines G et il est nettement appauvri en fibronectine. Ce produit est dépourvu ou ne contient que des doses infimes d'anticorps humains de groupe et est ainsi assimilable à un concentré de qualité isogroupe selon les normes préconisées par la Pharmacopée Européenne, ceci même en utilisant comme matériel d'origine un plasma non sélectionné par groupe sanguin. Il peut donc être très avantageusement utilisé en thérapeutique et tout particulièrement dans le traitement de l'hémophilie A qui nécessite des injections couramment répétées. Il peut également être utilisé comme réactif dans tout test ou analyse nécessitant un Facteur VIII de très haute pureté.

Les autres fractions séparées par cette colonne de chromatographie sont également intéressantes pour les protéines qu'elles contiennent, à savoir respectivement le fibrinogène, d'une part, qui est récupéré dans le premier filtrat, et d'autre part la fibronectine et le facteur von Willebrand qui sont élués par la première augmentation de la force ionique du tampon.

Les exemples suivants illustrent l'invention sans toutefois en limiter la portée.

### Exemple 1

### A)Prépurification et inactivation virale

On utilise comme matériau de départ un cryoprécipité de plasma humain, remis en suspension dans une solution aqueuse d'héparine sodique (à 2 U/ml) et contenant du Facteur VIII à une activité spécifique comprise entre 0.6 et 1.1 Ul/mg.

Le pH de la suspension est ajusté à 7-7,1 avec de l'acide acétique 0.1 M.

Cette suspension de cryoprécipité est soumise à une prépurification par traitement au gel d'alumine et précipitation à froid. On ajoute à la suspension de l'hydroxyde d'alumine (108 g d'Al(OH)₃ à 2% pour 1 kg de cryoprécipité) sous agitation pendant 5 minutes à température ordinaire. On ajuste le pH à 6,5-6,6 avec de l'acide acétique 0.1 M puis on refroidit la suspension à 14-16°C, sous agitation. Dès que la température voulue est atteinte, on centrifuge à 14-16 °C, on récupére le surnageant et on le stérilise par filtration.

Cette solution prépurifiée est soumise à un traitement d'inactivation virale par solvant-détergent, en présence de Tween®-TNBP, suivant la méthode décrite dans la demande de brevet européen n° 0 131 740.

### B) Séparation chromatographique sur Fractogel TSK-DEAE

On prépare une colonne de chromatographie avec de la résine Fractogel® TSK-DEAE 650 (M) (Merck). On prévoit une quantité de 0,5 L de Fractogel® par kg de cryoprécipité. La colonne est lavée par 5 volumes de solution de NaCl 0,1 M puis équilibrée avec le tampon suivant :
citrate de sodium trisodique (2,94 g/l), chlorure de calcium (1 mM), chlorure de sodium (0.11 M), glycocolle (9 g/l), et lysine (3 g/l).

La solution de cryoprécipité prépurifiée décrite au paragraphe A) est injectée sur la colonne.

Le filtrat et les éluats suivants sont récupérés et leur teneur en protéines est suivie par la mesure de l'absorption à 280 nm (notée ci-après D.O.).

Le premier filtrat montre un pic de protéines non adsorbées par la colonne qui correspond essentiellement au fibrinogène (voir exemple 3).

Après passage de ce pic, quand la D.O. est retombée à la ligne de base, on élue la colonne avec le même tampon dont on augmente une première fois la force ionique par addition de NaCl, à une concentration finale de 0,15 M. Ce tampon désorbe un pic de protéines contenant la plus grande partie du facteur von Willebrand et de la fibronectine (voir exemple 4).

Après passage de ce pic, quand la D.O. est retombée à la ligne de base, on augmente une deuxième fois la force ionique du tampon par addition de NaCl à la concentration finale de 0,25 M.

Dans ces conditions le Facteur VIII s'élue à une concentration de l'ordre de 30 à 40 UI/ml.

### Exemple 2 : Préparation du concentré de Facteur VIII

La solution de Facteur VIII obtenue par le procédé chromatographique décrit dans l'exemple 1 est suffisamment pure et concentrée pour être directement conditionnée en flacons et lyophilisée, sans étape supplémentaire d'ultrafiltration.

La concentration peut éventuellement être ajustée par dilution avec le même tampon d'élution pour ajuster l'activité spécifique par flacon selon des normes légales.

La composition moyenne des solutions récoltées est la suivante :

| | |
|---|---|
| Protéines (g/l) | 0,16 - 0,25 |
| Facteur VIII:C(UI/ml) | 30-45 |
| Activité spécifique du Facteur VIII:C (UI/mg) | 120 - 250 |
| Fibrinogène (g/l) | <0,1 |
| Facteur von Willebrand : Ag (U/ml) | 11 - 23 |
| Facteur von Willebrand : RCO (U/ml) | 10 - 20 |
| Facteur VIII:Ag (U/ml) | 35 - 60 |
| IgG (mg/ml) (Néphélémétrie) | <0,011 |
| Anticorps anti-A naturels et immuns | 0 - 2 |
| Fibronectine (mg/l) | 15 - 40 |

Après lyophilisation, la solubilité du produit est instantanée et le produit est limpide. Le taux de Facteur VIII:C est stable sur 24 heures à température ambiante.

Les injections sur l'homme indiquent une récupération du Facteur VIII:C comparable à celle obtenue avec les produits de moindre pureté. De même la demi-durée de vie est équivalente à celle des autres produits.

Ce concentré s'avère être un produit de très haute valeur thérapeutique, particulièrement adapté aux injections répétées nécessaires dans le traitement de l'hémophilie A.

### Exemple 3 : Purification d'un concentré de fibrinogène

Le premier filtrat de la chromatographie sur DEAE-Fractogel décrit dans l'exemple 1 contient principalement du fibrinogène mais aussi de l'albumine, des immunoglobulines et les agents d'inactivation virale (Tween® et TNBP).

Le fibrinogène est purifié à partir de cette solution par une nouvelle étape de chromatographie sur colonne de résine d'héparine-sépharose.

Cette chromatographie est effectuée dans le même tampon que la précédente, ajusté à 0,06 M en NaCl, ce qui permet d'éviter une dialyse entre les deux chromatographies.

Le filtrat de la première chromatographie est dilué pour obtenir une osmolarité de 280 mosM à un pH de 6,5 avant d'être injecté sur la deuxième colonne. On retrouve dans le second filtrat, l'albumine, les immunoglobulines, le Tween® et le TNBP. Le fibrinogène est adsorbé sur la colonne.

Quand la D.O. du filtrat est retombée au niveau de base, on élue la colonne avec le même tampon après avoir augmenté sa force ionique par addition de NaCl à une concentration finale de 0,16 M.

La fraction de fibrinogène recueillie est ensuite concentrée et dialysée sur un système de cassette. Le produit concentré est réparti en flacons et lyophilisé.

Ce fibrinogène concentré répond aux normes de qualité fixée par la Pharmacopée européenne.

En outre il peut servir de substrat pour la préparation de colle biologique selon la demande de brevet européenne EP-A-0 305 243

### Exemple 4 : Préparation de concentré de facteur von Willebrand

La fraction éluée de la chromatographie sur DEAE-Fractogel® en présence de 0,15 M de NaCl, décrite dans l'exemple 1, est fort enrichie en facteur von Willebrand et en fibronectine. Elle renferme encore du Tween® et du TNBP.

Cette fraction est diluée pour amener son osmolarité à 385-390 mosM avec le tampon de base de chromatographie (citrate trisodique, chlorure de calcium, lysine, glycine, pH 7, osmolarité 18 mosM).

Elle est ensuite injectée sur une deuxième colonne de DEAE-Fractogel®, équilibrée avec le même tampon que précédemment et contenant du NaCl à une concentration finale de 0,11 M, ajusté à pH 7 et 387 mosM.

Dans ces conditions on assure une très bonne élimination du Tween® et du TNBP.

La solution de départ étant déjà très concentrée en facteur von Willebrand, la fixation de celui-ci sur la colonne est nettement plus grande qu'au cours du passage sur la première colonne. La capacité de fixation de la colonne est d'au moins 160 U Ag/ml (unité d'antigène de facteur von Willebrand) ou de 100 U RCO/ml (unités de cofacteur de ristocétine).

L'élution de la fraction contenant le facteur von Willebrand s'effectue par addition au tampon de NaCl à une concentration finale de 0,15 M.

La solution de facteur von Willebrand éluée est suffisamment concentrée pour ne pas nécessiter d'ultrafiltration supplémentaire ; elle est répartie en flacons et lyophilisée.

Le concentré obtenu est de très haute pureté et présente une activité spécifique supérieure à 100 U RCO/mg. Il contient encore de la fibronectine mais celle-ci ne nuit pas à son activité.

### Exemple 5 : Préparation d'un concentré de fibronectine

On peut également préparer un concentré de fibronectine à partir du même éluat de départ que dans l'exemple 4.

On peut en effet séparer le facteur von Willebrand et la fibronectine sur la base de leur différence de poids moléculaires. Par tamisage moléculaire sur colonne de Séphacryl® S-400 (R) (Pharmacia) par exemple, on sépare une première fraction de haut poids moléculaire qui contient le facteur von Willebrand et ensuite une fraction qui contient la fibronectine qui peut être directement conditionnée et lyophilisée.

## Revendications

1. Procédé de séparation des protéines **F VIII, fibrinogène, fibronectine et facteur von Willebrand** du plasma humain ou animal et de préparation de concentrés desdites protéines à usage thérapeutique,
**caractérisé en ce que**
il comporte les étapes suivantes :
- on utilise comme matériau de départ la fraction du plasma cryoprécipitée,
- constituée essentiellement de fibrinogène, de fibronectine, de facteur von Willebrand et de Facteur VIII ;
- on soumet ledit cryoprécipité remis en solution aqueuse à une séparation unique par chromatographie sur une résine échangeuse d'anions dont la matrice est un gel de type polymère vinylique macroréticulé, capable de par ses propriétés de porosité et d'hydrophobicité de retenir le complexe Facteur VIII - facteur von Willebrand ;
- et on récupère sélectivement les différentes protéines par des augmentations successives de la force ionique du tampon d'élution.

2. Procédé selon la revendication 1, **caractérisé en ce que** le gel de type polymère vinylique est du Fractogel 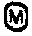 -TSK.

3. Procédé selon la revendication 1 ou 2, **caractérisé en ce que** le caractère échangeur d'anion de la résine est apporté par des groupements de type DEAE greffés sur la matrice.

4. Procédé selon l'une quelconque des revendications 1 à 3, **caractérisé en ce que** la fraction de départ contient du Facteur VIII pouvant présenter une activité spécifique supérieure on égale à 0.1 Ul/mg de protéines.

5. Procédé selon l'une quelconque des revendications 1 à 4, **caractérisé en ce que** la fraction de départ a subi un traitement de prépurification comprenant :
- un traitement à l'hydroxyde d'aluminium
- un refroidissement à 14-16 °C,
- une centrifugation et la récupération du surnageant.

6. Procédé selon l'une quelconque des revendications 1 à 5, **caractérisé en ce que** la chromatographie est effectuée avec un tampon qui contient de la lysine et du glycocolle et dont on augmente la force ionique à l'aide de chlorure de sodium en concentrations croissantes.

7. Procédé selon la revendication 6, **caractérise en ce que** le tampon contient de 2 à 4 g/l de lysine et de 8 à 11 g/l de glycocolle.

8. Procédé selon l'une quelconque des revendications 1 à 7, **caractérisé en ce que** la concentration en chlorure de sodium du tampon :
- est de 0.11 M pour l'équilibrage de la colonne et la charge de l'échantillon, ce qui permet l'adsorption de la fibronectine, du facteur von Willebrand et du Facteur VIII et laisse passer le fibrinogène dans le filtrat ;
- est augmentée à 0.15 M pour éluer la fibronectine et la plus grande partie du facteur von Willebrand ;
- est augmentée à 0,25 M pour éluer le Facteur VIII.

9. Procédé selon l'une quelconque des revendications 1 à 8, **caractérisé en ce qu'**on effectue un traitement d'inactivation virale en présence d'agents chimiques d'inactivation sur la fraction de plasma juste avant de la soumettre à l'étape de séparation chromatographique.

10. Procédé selon l'une quelconque des revendications 1 à 9, **caractérisé en ce qu'**il comprend la récupération du filtrat de la chromatographie et son passage sur une chromatographie sur résine d'héparine-sépharose pour récupérer un concentré de fibrinogène.

11. Procédé selon l'une quelconque des revendications 1 à 9, **caractérisé en ce qu'**il comprend la récupération du premier éluat de la chromatographie et son passage sur une deuxième colonne identique à la première, avec le même tampon ajusté à 0.15 M en de chlorure de sodium, pour récupérer un concentré de facteur von Willebrand.

12. Procédé selon l'une quelconque des revendications 1 à 9, **caractérisé en ce qu'**il comprend la récupération du premier éluat de la chromatographie et son passage sur une chromatographie de tamisage moléculaire, pour récupérer un concentré de fibronectine.

13. Concentré de Facteur VIII susceptible d'être obtenu par le procédé selon l'une quelconque des revendications 1 à 9, **caractérisé en ce qu'**il présente une activité spécifique au moins égale à 100 UI/mg de protéines et qu'il est de qualité assimilable à celle d'un concentré isogroupe.

## Patentansprüche

1. Verfahren zur Trennung von Proteinen Faktor VIII, Fibrinogen, Fibronectin und von Willebrand-Faktor des menschlichen oder tierischen Plasmas und zur Herstellung von Konzentraten dieser Proteine zum therapeutischen Gebrauch,
**dadurch gekennzeichnet, daß** es die folgenden Schritte aufweist :
- als Ausgangsmaterial wird die bei niedriger Temperatur gefällte Plasmafraktion, die im wessentlichen aus Fibrinogen, Fibronectin, von Willebrand-Faktor und Faktor VIII besteht, vermendet;
- das besagte bei niedriger Temperatur gefällte, das wieder in wäßrige Lösung gebracht wurde, wird einer einzigen Trennung durch Chromatogaphie auf einem Anionenaustauscherharz unterworfen, dessen Matrix ein Gel von der Art eines makroreticularen Vinylpolymeren ist, das durch seine Porositäts- und Hydrophobieeigenshaften fähig ist, den Komplex aus Faktor VIII und von Willebrand-Faktor zuruckzuhalten ;
- durch aufeinanderfolgende Erhöhungen der Ionenstärke des Elutionspuffers werden selektiv die verschiedenen Proteine gewonnen.

2. Verfahren nach Anspruch 1, **dadurch gekennzeichnet, daß** das Gel von der Art des Vinylpolymeren Fractogel 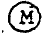 -TSK ist.

3. Verfahren nach Anspruch 1 oder 2, **dadurch gekennzeichnet, daß** der Anionen austauschende Charakter des Harzes von auf die Matrix gepfropften Gruppen vom DEAE-Typ herrührt.

4. Verfahren nach einem der Ansprüche 1 bis 3, **dadurch gekennzeichnet, daß** die Ausgangsfraktion Faktor VIII enthält, der eine spezifische Aktivität größer oder gleich 0,1 I.E. pro mg an Proteinen aufweisen kann.

5. Verfahren nach einem der Ansprüche 1 bis 4, **dadurch gekennzeichnet, daß** die Ausgangsfraktion eine Vorreinigungsbehandlung erfahren hat, die
- eine Behandlung mit Aluminiumhydroxid,
- eine Abkühlung auf 14-16°C,
- ein Zentrifugieren und die Wiedergewinnung des Überstands umfaßt.

6. Verfahren nach einem der Ansprüche 1 bis 5, **dadurch gekennzeichnet, daß** die Chromatographie mit einem Puffer durchgeführt wird, der Lysin und Glycin enthält und dessen Ionenstärke mit Hilfe von Natriumchlorid in zunehmender Konzentration erhöht wird.

7. Verfahren nach Anspruch 6, **dadurch gekennzeichnet, daß** der Puffer 2 bis 4 g/l Lysin und 8 bis 11 g/l Glycin enthält.

8. Verfahren nach einem der Ansprüche 1 bis 7, **dadurch gekennzeichnet, daß** die Natriumchlorid-Konzentration des Puffers
- bei der Äquilibrierung des Kolonne und beim Beladen der Probe 0,11 M ist, was die Adsorption des Fibronectins, des von Willebrand-Faktors und des Faktors VIII erlaubt und das Fibrinogen in das Filtrat durchlaufen läßt,
- auf 0,15 M erhöht wird, um das Fibronectin und den größten Teil des von Willebrand-Faktors zu eluieren,
- auf 0,25 M erhöht wird, um den Faktor VIII zu eluieren.

9. Verfahren nach einem der Ansprüche 1 bis 8, **dadurch gekennzeichnet, daß** mit der Plasmafraktion, bevor sie dem Schritt der chromatographischen Trennung unterworfen wird, eine Behandlung zur Virusinaktivierung in Gegenwart von chemischen Inaktivierungsagentien durchgeführt wird.

10. Verfahren nach einem der Ansprüche 1 bis 9, **dadurch gekennzeichnet, daß** es das Auffangen des Filtrats der Chromatographie und dessen Lauf über eine Chromatographie über Heparin-Sepharose-Harz einschließt, um ein Fibrinogen-Konzentrat zu gewinnen.

11. Verfahren nach einem der Ansprüche 1 bis 9, **dadurch gekennzeichnet, daß** es das Auffangen des ersten Eluats der Chromatographie und dessen Lauf über eine zweite, mit der ersten identische Kolonne einschließt, mit dem gleichen, auf 0,15 M eingestellten Natriumchlorid-Puffer, um ein Konzentrat des von Willebrand-Faktors zu gewinnen.

12. Verfahren nach einem der Ansprüche 1 bis 9, **dadurch gekennzeichnet, daß** es das Auffangen des ersten Eluats der Chromatographie und dessen Lauf über eine Molekularsieb-Chromatographie einschließt, um ein Fibronectin-Konzentrat zu gewinnen.

13. Konzentrat von Faktor VIII, erhältlich durch das Verfahren nach einem der Ansprüche 1 bis 9, **dadurch gekennzeichnet, daß** es eine spezifische Aktivität von wenigstens 100 I.E. pro mg an Proteinen besitzt und daß es von einer Qualität vergleichbar mit der eines Konzentrats gleicher Blutgruppe ist.

## Claims

1. Process for the separation of FVIII, fibrinogen, fibronectin and von Willebrand's factor proteins of human or animal plasma and for the preparation of concentrates of said proteins for therapeutic use,
**characterised in that** it includes the following steps :
- the cryoprecipitated fraction of plasma is used as starting material,
- which essentially consists of fibrinogen, fibronectin,von Willebrand's factor and FVIII ;
- said cryoprecipitate, dissolved in aqueous solution, is subjected to a single separation by chromatography on an anion exchange resin of which the matrix is a gel of the macroreticular vinyl polymer type which, as a result of its hydrophobic and porous properties, is capable of retaining the Factor VIII-von Willebrand's factor complex ;
- and the different proteins are recovered selectively by successively increasing the ionic strength of the elution buffer.

2. Process according to claim 1, **characterised in that** the vinyl polymer-type gel is Fractogel 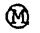 -TSK.

3. Process according to claim 1 or 2, **characterised in that** the anion exchange character of the resin is provided by groups of the DEAE type grafted onto the matrix.

4. Process according to any one of claims 1 to 3,
**characterised in that** the starting fraction contains Factor VIII capable of having a specific activity greater than or equal to 0.1 IU/mg protein.

5. Process according to any one of claims 1 to 4,
**characterised in that** the starting fraction has been subjected to a prepurification treatment comprising :
- treatment with aluminium hydroxide,
- cooling to 14-16°C,
- centrifuging and recovering the supernatant.

6. Process according to any one of claims 1 to 5, **characterised in that** the chromatography is carried out with a buffer that contains lysine and glycine and of which the ionic strength is increased by means of sodium chloride in increasing concentrations.

7. Process according to claim 6, **characterised in that** the buffer contains from 2 to 4 g/l of lysine and from 8 to 11 g/l of glycine.

8. Process according to any one of claims 1 to 7,
**characterised in that** the concentration of sodium chloride in the buffer :
- is 0.11 M for the equilibration of the column and the sample charge, which permits the adsorption of fibronectin, Willebrand's factor and Factor VIII and allows the fibrinogen to pass through in the eluate;
- is increased to 0.15 M in order to elute the fibronectin and the greater portion of Willebrand's factor;
- is increased to 0.25 M in order to elute Factor VIII.

9. Process according to any one of claims 1 to 8, **characterised in that** a virus inactivation treatment in the presence of chemical inactivation agents is carried out on the plasma fraction immediately prior to subjecting it to the chromatographic separation step.

10. Process according to any one of claims 1 to 9, **characterised in that** it comprises the recovery of the chromatography eluate and its chromatography on heparin-Sepharose resin in order to recover a fibrinogen concentrate.

11. Process according to any one of claims 1 to 9, **characterised in that** it comprises recovering the first eluate of the chromatography and passing it over a second column that is identical to the first, with the same buffer adjusted to 0.15 M sodium chloride, in order to recover a concentrate of Willebrand's factor.

12. Process according to any one of claims 1 to 9, **characterised in that** it comprises recovering the first eluate of the chromatography and subjecting it to molecular sieve chromatography in order to recover a fibronectin concentrate.

13. Concentrate of Factor VIII capable of being obtained by the process according to any one of claims 1 to 9, **characterised in that** it has a specific activity at least equal to 100 IU/mg protein and that it is of a quality comparable to that of a single-group concentrate.
